# EUROPEAN PATENT APPLICATION

(11) **EP 4 641 199 A2**
(43) Date of publication of application: **29.10.2025**
(21) Application number: 23910364.1
(22) Date of filing: 22.12.2023
(51) Int. Cl.: G01N 33/558, G01N 33/58, G01N 33/543, G01N 33/569, G01N 33/68

(54) **MULTI-JOINT TEST QUANTUM DOT QUANTITATIVE TEST CASSETTE, PREPARATION METHOD AND DEVICE THEREFOR, AND USE METHOD THEREFOR**

(30) Priority: 26.12.2022 CN 202211682483; 26.12.2022 CN 202211676895
(71) Applicant: Koch Biotechnology (Beijing) Co., Ltd., Beijing 102600 (CN)
(72) Inventor: HOU, Dezhi, Beijing 102600 (CN); SONG, Yu, Beijing 102600 (CN)
(74) Representative: Chung, Hoi Kan
(86) International application number: PCT/CN2023/140846
(87) International publication number: WO 2024/140438

(57) **Abstract**

Disclosed is a multiplex quantum dot-based quantitative test card, and a preparation method, apparatus, and use method thereof in the technical field of biological testing, the multiplex quantum dot-based quantitative test card including a multiplex card housing and a test strip, where the multiplex card housing is provided with a plurality of reaction slots configured to accommodate the test strip; the multiplex card housing further includes a plurality of sample loading ports and a plurality of observation windows, where the sample loading ports and the observation windows are in one-to-one correspondence with the reaction slots; and the test strip includes a conjugate pad coated with quantum dot-labeled antibodies, where the quantum dot-labeled antibodies serve as chromogenic substances. By simultaneously employing quantum dot-based quantitative testing, a plurality of test lines on a nitrocellulose membrane, and a multiplex card housing, the multiplex quantum dot-based quantitative test card provided in the present application addresses both quantitative and multiplex testing challenges, thereby enhancing testing efficiency and accuracy; furthermore, it enables rapid diagnosis for various allergic diseases caused by inhaled and ingested allergens in humans.

## Description

The present application claims priority to Chinese Patent Application No. 202211682483.9, filed with the China National Intellectual Property Administration on December 26, 2022 and entitled "MULTIPLEX QUANTUM DOT-BASED QUANTITATIVE TEST CARD, AND PREPARATION METHOD THEREOF", and Chinese Patent Application No. 202211676895.1, filed with the China National Intellectual Property Administration on December 26, 2022 and entitled "MULTIPLEX TESTING APPARATUS, AND USE METHOD THEREOF", which are incorporated herein by reference in their entirety.

### TECHNICAL FIELD

The present application relates to the technical field of biological testing, and in particular, to a multiplex quantum dot-based quantitative test card, and a preparation method, apparatus, and use method thereof.

### BACKGROUND

At present, the prevalence of allergic diseases is increasing, with approximately 300-400 million patients suffering from allergic diseases in China, and the number continues to rise annually. The diagnosis of allergic diseases mainly includes *in vivo* and *in vitro* allergen testing. *In vitro* testing has garnered growing attention due to its high safety, readily available formulations, independence from patients' medication, and capability of simultaneously detecting multiple allergens, particularly suitable for pediatric patients. The testing of allergen-specific IgE (sIgE) antibodies plays a critical role in reflecting disease status, aiding diagnosis, and predicting disease onset and prognosis, and has been incorporated into multiple clinical practice guidelines. IgE test results can be classified into quantitative and qualitative types, where qualitative results only indicate negative or positive outcomes, such as those obtained via immunoblotting methods; in contrast, fully quantitative allergen testing can measure the specific level of IgE antibodies in serum, providing more intuitive numerical results that can reflect disease correlation, more effectively predict disease progression and prognosis, and hold greater clinical significance in diagnosing allergic diseases. Moreover, quantitative testing exhibits superior precision, repeatability, and more reliable test results.

Existing methods for the quantitative testing of allergen-specific antibodies, such as enzyme-linked immunosorbent assay (ELISA), suffer from prolonged test time, complex operational procedures, and reliance on large-scale equipment. Consequently, fluorescent immunoassay technology, which utilizes fluorescently labeled antibodies or antigen molecules to quantitatively analyze test samples by detecting changes in fluorescence signal intensity following specific binding, is more suitable for the testing of allergen-specific IgE antibody levels. Quantum dots (QDs), as a novel class of fluorescent nanocrystals, exhibit high fluorescence efficiency, photobleaching resistance, long fluorescence lifetime, narrow and symmetrical emission spectra, single-wavelength excitation with multiplexed emissions, and capability of simultaneous multi-analyte testing. In QD microsphere labels, the QDs are encapsulated within microspheres, enabling fluorescence signal amplification of the QDs. The encapsulation layer can not only stabilize the QDs and enhance the microspheres' biocompatibility and colloidal stability, but also enable functionalization with diverse groups on the microsphere surface to facilitate conjugation between the microsphere surface and various labels. These superior characteristics of QDs make them an optimal choice for preparing immunofluorescence probes, thereby enabling the quantitative testing of allergen-specific IgE antibodies in test samples through fluorescent immunoassay technology.

Current commercially available products for quantitative immunochromatographic testing of allergens face challenges in performing simultaneous multiplex testing and suffer from severe interference during multiplex testing. Consequently, there exists an urgent need for an allergen-targeted quantitative immunochromatographic testing product capable of simultaneous multiplex testing.

### SUMMARY

The present application provides a multiplex quantum dot-based quantitative test card, and a preparation method, apparatus, and use method thereof, aiming to solve the problems that current commercially available products for quantitative immunochromatographic testing of allergens face challenges in performing simultaneous multiplex testing and suffer from severe interference during multiplex testing.

A first aspect of embodiments of the present application provides a multiplex quantum dot-based quantitative test card, where the test card includes:
a multiplex card housing and a test strip, where
the multiplex card housing is provided with a plurality of reaction slots configured to accommodate the test strip;
the multiplex card housing further includes a plurality of sample loading ports and a plurality of observation windows, where the sample loading ports and the observation windows are in one-to-one correspondence with the reaction slots; and
the test strip includes a conjugate pad coated with quantum dot-labeled antibodies, where
the quantum dot-labeled antibodies serve as chromogenic substances.

Optionally, the test strip further includes a sample pad, a nitrocellulose membrane, an absorbent pad, and a backing plate, where
the sample pad, the conjugate pad, the nitrocellulose membrane, and the absorbent pad are sequentially and uniformly adhered to the backing plate;
the nitrocellulose membrane is coated with a plurality of test lines and a control line; and
the plurality of test lines and the control line are uniformly spaced and immobilized on the nitrocellulose membrane.

Optionally, the plurality of test lines are coated with distinct allergen antigens, while the control line is coated with goat anti-chicken IgY antibodies.

Optionally, the allergen antigens include at least any one selected from the group consisting of: cat dander, mugwort, birch trees, timothy grass, cockroaches, dog dander, plantains, ragweed, house dust mites, *Alternaria alternata* (inhaled allergens), peanuts, milk, codfish, scallops, chicken egg white, shrimp, crabs, soybeans, wheat, and almonds (ingested allergens).

Optionally, the quantum dot-labeled antibodies coated on the conjugate pad are mouse anti-human IgE antibodies and chicken IgY antibodies.

A second aspect of embodiments of the present application provides a preparation method of a multiplex quantum dot-based quantitative test card, where the preparation method includes the following steps:
preparing a coated nitrocellulose membrane containing a plurality of test lines and a control line;
preparing a conjugate pad coated with two types of quantum dot-labeled antibodies, where the two types of quantum dot-labeled antibodies include: quantum dot-labeled mouse anti-human IgE antibodies and quantum dot-labeled chicken IgY antibodies; and
assembling the coated nitrocellulose membrane and the conjugate pad coated with the two types of quantum dot-labeled antibodies to obtain the multiplex quantum dot-based quantitative test card.

Optionally, the step of preparing the coated nitrocellulose membrane containing the plurality of test lines and the control line includes:
fixedly adhering a nitrocellulose membrane to a central portion of a backing plate; and
immobilizing allergen antigens and antibodies diluted to desired coating concentrations onto the nitrocellulose membrane, and performing drying to obtain the coated nitrocellulose membrane.

Optionally, the step of preparing the conjugate pad coated with the two types of quantum dot-labeled antibodies includes:
obtaining a quantum dot solution;
preparing the quantum dot-labeled mouse anti-human IgE antibodies and the quantum dot-labeled chicken IgY antibodies; and
mixing the quantum dot-labeled mouse anti-human IgE antibodies and the quantum dot-labeled chicken IgY antibodies in proportion, immobilizing a resulting mixture onto the conjugate pad, and performing drying to obtain the conjugate pad coated with the two types of quantum dot-labeled antibodies.

Optionally, the step of assembling the coated nitrocellulose membrane and the conjugate pad coated with the two types of quantum dot-labeled antibodies includes:
assembling the coated nitrocellulose membrane, the conjugate pad coated with the two types of quantum dot-labeled antibodies, a sample pad, an absorbent pad, and the backing plate to obtain a test strip; and
fixedly positioning the test strip within an accommodation slot of a multiplex card housing to obtain the multiplex quantum dot-based quantitative test card.

Optionally, the step of assembling the coated nitrocellulose membrane, the conjugate pad coated with the two types of quantum dot-labeled antibodies, the sample pad, the absorbent pad, and the backing plate to obtain the test strip includes:
fixedly adhering the absorbent pad to an edge, proximal to the control line, of the coated nitrocellulose membrane, and fixedly adhering the conjugate pad coated with the two types of quantum dot-labeled antibodies to an edge, proximal to the test lines, of the coated nitrocellulose membrane;
fixedly adhering the sample pad to an edge, distal to the test lines, of the conjugate pad coated with the two types of quantum dot-labeled antibodies; and
cutting the backing plate with the coated nitrocellulose membrane, the absorbent pad, the conjugate pad coated with the two types of quantum dot-labeled antibodies, and the sample pad fixedly adhered thereto into a strip of 2-6 mm to obtain the test strip.

The testing principle of the multiplex quantum dot-based quantitative test card is as follows: IgE antibodies in a test sample bind to the quantum dot-labeled mouse anti-human IgE antibodies (chromogenic substances) and migrate along the test strip toward the test lines and the control line, where they form an "allergen antigen-sIgE-mouse anti-human IgE quantum dot" complex upon binding to the distinct allergen antigens coated on the test lines. Upon excitation by an excitation light source, the quantum dots emit fluorescent signals. The higher the concentration of sIgE, the larger the quantity of quantum dots accumulated on the test lines and the stronger the excited fluorescence signals. By measuring the intensity of the signals, the level of IgE antibodies in the test sample can be calculated to achieve the purpose of quantitative testing. When the quantum dot-labeled chicken IgY antibodies (chromogenic substances) migrate to the control line, a "goat anti-chicken IgY-chicken IgY quantum dot" complex is formed, generating a signal. If no fluorescence signal appears at the control line, it indicates reaction system failure. The intensity of fluorescence signals at the test lines is directly proportional to the level of specific IgE antibodies in the test sample, and by comparing the intensity of fluorescence signals against a standard curve, the level of specific IgE antibodies in the test sample can be obtained.

The present application has the following advantages:
1) by simultaneously employing quantum dot-based quantitative testing, a plurality of test lines on a nitrocellulose membrane, and a multiplex card housing, the present application addresses both quantitative and multiplex testing challenges, thereby enhancing testing efficiency and accuracy;
2) the present application achieves the technical objective of rapid diagnosis for various allergic diseases caused by inhaled and ingested allergens in humans;
3) the present application improves the chromatographic reaction effect of the test strips while reducing interference during concurrent testing of the plurality of test strips, providing advantages of user-friendly operation, high sensitivity, and clinically actionable quantitative testing results for more accurate and effective medical diagnosis; and
4) the blocking solution used during the labeling of antibodies with quantum dots in the present application is conducive to solving the problems of cross-reactivity between test items and nonspecific interference in the test sample.

The present application provides a multiplex testing apparatus and a use method thereof, aiming to solve the problems that current commercially available colloidal gold-based immunochromatographic products face challenges in performing multiplex testing with a single sample loading operation and suffer from severe interference between multiplex test items.

A third aspect of embodiments of the present application provides a multiplex testing apparatus, including: a test card and a test strip adapted for use with the test card, where
the test card includes a first housing and a second housing, where the second housing is provided with two accommodation slots configured to accommodate the test strip;
the first housing is provided with a sample loading port and a flow dividing chamber disposed at a position where the sample loading port is located, where the flow dividing chamber includes a flow divider positioned in center alignment with the sample loading port; and
the flow dividing chamber includes two outlet orifices respectively disposed at two sides of the flow divider, where the two outlet orifices are in fluid communication with the two accommodation slots.

Optionally, vent holes are formed in both side walls of the first housing and positioned respectively in correspondence with the two outlet orifices.

Optionally, the first housing is provided with two observation windows, where
the two observation windows are arranged respectively in one-to-one correspondence with the two accommodation slots.

Optionally, the test strip includes a first end and a second end, where when the test strip is positioned within the accommodation slots, the first end is positioned in correspondence with the outlet orifices; and
the test strip includes a backing plate, and a sample pad, a colloidal gold pad, a nitrocellulose membrane, and an absorbent pad sequentially disposed on the backing plate along a direction extending from the first end to the second end, where
the nitrocellulose membrane is coated with a plurality of test lines and a control line.

Optionally, the nitrocellulose membrane is positioned in correspondence with the observation windows.

Optionally, the plurality of test lines are coated with multiple types of allergen antigens, while the control line is coated with goat anti-chicken IgY antibodies; and
each of the test lines is coated with a distinct one of the multiple types of allergen antigens.

Optionally, the multiple types of allergen antigens include inhaled allergen antigens and ingested allergen antigens, where
the inhaled allergens include one selected from the group consisting of: cat dander, mugwort, birch trees, timothy grass, cockroaches, dog dander, plantains, ragweed, house dust mites, and *Alternaria alternata;* and
the ingested allergens include one selected from the group consisting of: peanuts, milk, codfish, scallops, chicken egg white, shrimp, crabs, soybeans, wheat, and almonds.

Optionally, antibodies coated on the colloidal gold pad are colloidal gold particle-labeled mouse anti-human IgE antibodies and colloidal gold particle-labeled chicken IgY antibodies.

A fourth aspect of embodiments of the present application provides a use method of a multiplex testing apparatus, the multiplex testing apparatus including a test card and a test strip, where the test card includes a sample loading port, and the use method includes:
loading a test sample into the sample loading port; and
determining test information generated by the test strip in response to the test sample.

Optionally, the test sample is a whole blood sample, a plasma sample, or a serum sample.

The present application has the following beneficial effects:
1) the present application achieves the technical objective of rapid detection of allergens (including inhaled or ingested allergens) causing various allergic reactions in humans, enabling precise allergen identification to facilitate physicians' rapid diagnosis and patients' self-testing. The single-port/single-sample-loading design provides operational convenience by eliminating the need for multiple sample collections, and the testing apparatus exhibits high sensitivity and portability without requiring ancillary instrumentation, i.e., all operations are performable according to the instructions for use;
2) the present application provides a special test card housing configured for simultaneous testing with two test strips through a single sample loading operation, thereby overcoming the limitation of conventional multiplex test card housings that require multiple sample loading operations; and
3) the present application can be applied to whole blood testing, especially suitable for infants and young children as well as self-testing at home.

### BRIEF DESCRIPTION OF THE DRAWINGS

To describe the technical solutions in the embodiments of the present application more clearly, the following briefly describes the accompanying drawings required for describing the embodiments. Apparently, the accompanying drawings in the following description show merely some embodiments of the present application, and a person of ordinary skill in the art may still derive other drawings from these accompanying drawings without making creative efforts.
FIG. 1 is a schematic structural diagram of a multiplex quantum dot-based quantitative test card according to an embodiment of the present application;
FIG. 2 is a schematic structural diagram of a test strip of a multiplex quantum dot-based quantitative test card according to an embodiment of the present application;
FIG. 3 is a graph showing correlation test results of levels of allergen-specific IgE antibodies in a serum sample according to an embodiment of the present application;
FIG. 4 is a schematic structural diagram of a test card of a multiplex testing apparatus according to an embodiment of the present application;
FIG. 5A is a sectional view of a sample loading port in the direction of A-A in FIG. 4;
FIG. 5B is a partial enlarged view of FIG. 5A;
FIG. 6A is a schematic structural diagram of a first housing of the test card in the direction of B-B in FIG. 4;
FIG. 6B is a partial enlarged view of FIG. 6A;
FIG. 7 is a front perspective view of FIG. 4;
FIG. 8 is a schematic structural diagram of a test strip of a multiplex testing apparatus according to an embodiment of the present application; and
FIG. 9 is a flow chart of a use method of a multiplex testing apparatus according to an embodiment of the present application.

### DETAILED DESCRIPTION OF THE EMBODIMENTS

The technical solutions in the embodiments of the present application are clearly and completely described below with reference to the accompanying drawings in the embodiments of the present application. Apparently, the described embodiments are only some rather than all of the embodiments of the present application. All other embodiments obtained by a person of ordinary skill in the art based on the embodiments of the present application without making creative efforts shall fall within the protection scope of the present application.

### Example I

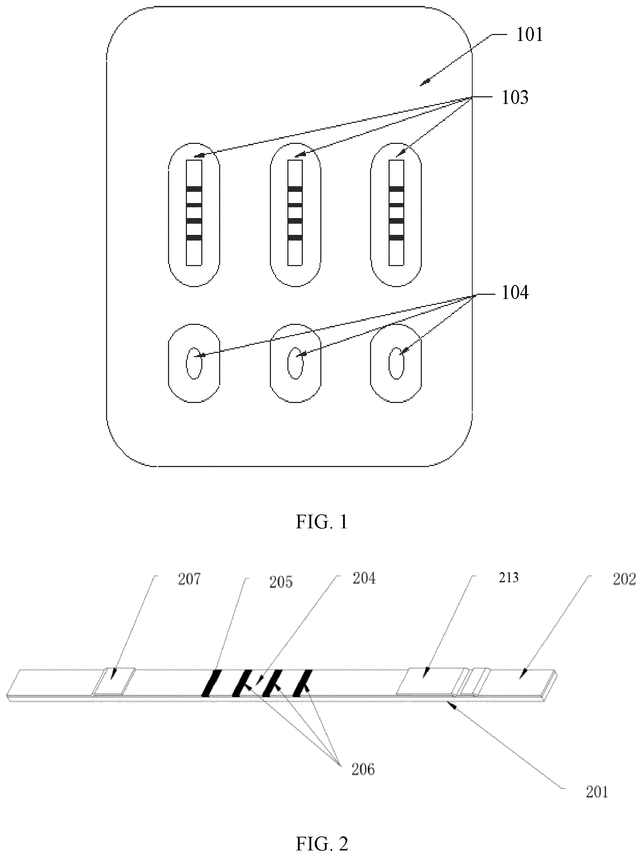

FIGs. 1-2 show a multiplex quantum dot-based quantitative test card 100 and a test strip 200 provided in this example.

FIG. 1 shows the multiplex quantum dot-based quantitative test card 100 provided in this example.

The multiplex quantum dot-based quantitative test card 100 includes a multiplex card housing 101 and a plurality of test strips 200.

The multiplex card housing 101 is provided with a plurality of accommodation slots (not shown) configured to accommodate the test strips 200.

Meanwhile, the multiplex card housing 101 further includes a plurality of sample loading ports 104 and a plurality of observation windows 103, where the sample loading ports are configured for loading of test samples and the observation windows are configured for observation of the test strips.

Preferably, a buffer zone (not marked) is arranged around the sample loading port 104. Since the flow of the test sample and the sample diluent requires a certain amount of time, the buffer zone is configured to temporarily retain the test sample loaded through the sample loading port and the sample diluent.

Herein, the sample loading ports 104, the observation windows 103, and the reaction slots are arranged in one-to-one correspondence to facilitate the arrangement of the test strips.

Preferably, the multiplex card housing is correspondingly provided with three accommodation slots, three sample loading ports, and three observation windows.

Preferably, the multiplex quantum dot-based quantitative test card is sealed and stored in an aluminum foil pouch containing a desiccant.

FIG. 2 shows the test strip 200 of the multiplex quantum dot-based quantitative test card provided in this example.

The test strip 200 includes a conjugate pad 213 coated with quantum dot-labeled antibodies.

Herein, the quantum dot-labeled antibodies serve as chromogenic substances.

Meanwhile, the test strip 200 further includes a sample pad 202, a nitrocellulose membrane 204, an absorbent pad 207, and a backing plate 201, where
the sample pad 202, the conjugate pad 213, the nitrocellulose membrane 204, and the absorbent pad 207 are sequentially and uniformly adhered to the backing plate 201.

Preferably, the sample pad 202 is a polyester fiber membrane or a glass fiber membrane.

Preferably, the conjugation pad 213 is a glass fiber membrane.

Preferably, the backing plate 201 is a PVC plate.

Preferably, the nitrocellulose membrane 204 is coated with a plurality of test lines 206 and a control line 205. During use, the test lines are configured to indicate test results of the multiplex quantum dot-based quantitative test card, while the control line is configured to indicate validity of the test results.

Herein, the sample pad is positioned in correspondence with the sample loading port to enable direct flow of the test sample to the sample pad, thereby reducing sample loss; and the nitrocellulose membrane is positioned in correspondence with the observation window to facilitate the detection of fluorescence signals from both the test line and the control line.

Herein, the plurality of test lines 206 and the control line 205 are uniformly spaced and immobilized on the nitrocellulose membrane. In this example, the test lines are provided in a total of three, where the three test lines and the control line are uniformly spaced at an interval of 2-6 mm (e.g., 2 mm, 3 mm, or 6 mm) along a lengthwise direction of the test strip, while the control line is arranged proximal to the absorbent pad.

Herein, the three test lines are coated with distinct allergen antigens, while the control line is coated with goat anti-chicken IgY antibodies. The allergen antigens coated on the test lines can bind to quantum dot-labeled antibodies (chromogenic substances) to form complexes, where the complexes emit detectable fluorescence signals upon laser excitation, and the measured fluorescence signal values are compared against a standard curve to calculate levels of IgE antibodies in the test sample; the goat anti-chicken IgY antibodies coated on the control line can bind to the quantum dot-labeled antibodies to form complexes, and the presence or absence of signals from the complexes indicates whether the test results of the multiplex quantum dot-based quantitative test card are valid.

Preferably, the standard curve is established by: preparing serial dilutions of an international standard for total IgE bodies at concentrations of 0.1 IU/mL, 0.3 IU/mL, 1.2 IU/mL, 4.8 IU/mL, 19.2 IU/mL, 38.3 IU/mL, 76.6 IU/mL, and 100 IU/mL; measuring corresponding signal values of 0.0016, 0.0049, 0.0199, 0.0754, 0.2761, 0.5004, 0.8845, and 1.1053 for each of the concentrations; and performing four-parameter fitting by substitution of the data to obtain the standard curve represented by the formula *y* = (*A* - *D*)/[1+(*x*/*C*)^*B*]+*D*, where A = 9.67167, B = -0.89759, C = 980.14709, D = -0.00171, and R² = 0.99995.

Preferably, the international standard for total IgE bodies is the International Standard Immunoglobulin E (IgE), human serum (NIBSC code: 11/234).

Preferably, each of the test lines is coated with a distinct one of the multiple types of allergen antigens.

Preferably, the multiple types of allergen antigens include any one selected from the group consisting of: cat dander, mugwort, birch trees, timothy grass, cockroaches, dog dander, plantains, ragweed, house dust mites, *Alternaria alternata* (inhaled allergens), peanuts, milk, codfish, scallops, chicken egg white, shrimp, crabs, soybeans, wheat, and almonds (ingested allergens).

For example, the plurality of test lines include a first test line, a second test line, and a third test line, where the first test line is coated with a crude extract antigen of peanuts; the second test line is coated with a crude extract antigen of cockroaches; and the third test line is coated with a crude extract antigen of soybeans. Those skilled in the art may make adjustments as needed, which will not be elaborated herein.

Preferably, the quantum dot-labeled antibodies coated on the conjugate pad are mouse anti-human IgE antibodies and chicken IgY antibodies.

As shown in Table 1, the test results of the multiplex quantum dot-based quantitative test card for simultaneous testing of multiple distinct inhaled and ingested allergens indicate that the multiplex quantum dot-based quantitative test card can simultaneously detect levels of IgE antibodies against distinct allergens in test samples. Ten replicate tests demonstrated high precision, thereby ensuring accuracy of the test results of the multiplex quantum dot-based quantitative test card. Therefore, by simultaneously employing quantum dot-based quantitative testing, a plurality of test lines on a nitrocellulose membrane, and a multiplex card housing, the present application addresses both quantitative and multiplex testing challenges, thereby enhancing testing efficiency and accuracy of the multiplex quantum dot-based quantitative test card.

**Table 1 Test Results of Levels of Allergen-specific IgE Antibodies in Serum Samples (IU/mL)**

| | Precision | | | | | | | | |
|---|---|---|---|---|---|---|---|---|---|
| | Cat dande r | Mugwo rt | Timoth y grass | Cockroac h | Dog dande r | Plantai n | Ragwee d | Hous e dust mite | *Alternari a alternata* |
| Result 1 | 0.43 | 2.42 | 3.52 | 11.24 | 1.53 | 13.23 | 4.29 | 1.65 | 6.45 |
| Result 2 | 0.44 | 1.98 | 3.91 | 9.02 | 1.32 | 10.56 | 3.46 | 1.83 | 6.32 |
| Result 3 | 0.51 | 2.21 | 3.81 | 10.03 | 1.48 | 11.73 | 3.85 | 1.84 | 5.69 |
| Result 4 | 0.47 | 2.14 | 4.25 | 9.76 | 1.42 | 11.43 | 3.74 | 1.81 | 5.16 |
| Result 5 | 0.49 | 2.37 | 3.17 | 10.87 | 1.55 | 12.75 | 4.16 | 1.75 | 5.92 |
| Result 6 | 0.55 | 1.86 | 3.5 | 8.2 | 1.34 | 9.51 | 3.17 | 2.02 | 5.29 |
| Result 7 | 0.52 | 2.01 | 4.42 | 9.01 | 1.39 | 10.5 | 3.47 | 1.92 | 6.1 |
| Result 8 | 0.42 | 2.42 | 3.45 | 11.26 | 1.52 | 13.26 | 4.30 | 1.64 | 5.94 |
| Result 9 | 0.47 | 1.96 | 4.18 | 8.86 | 1.33 | 10.35 | 3.41 | 1.88 | 6.29 |
| Result 10 | 0.48 | 2.33 | 3.862 | 10.69 | 1.52 | 12.54 | 4.09 | 1.75 | 5.85 |
| Mean value | 0.478 | 2.17 | 3.8072 | 9.894 | 1.44 | 11.59 | 3.79 | 1.81 | 5.90 |
| Precision | 8.64% | 9.65% | 10.49% | 11.06% | 6.30% | 11.47% | 10.70% | 6.49 % | 7.23% |

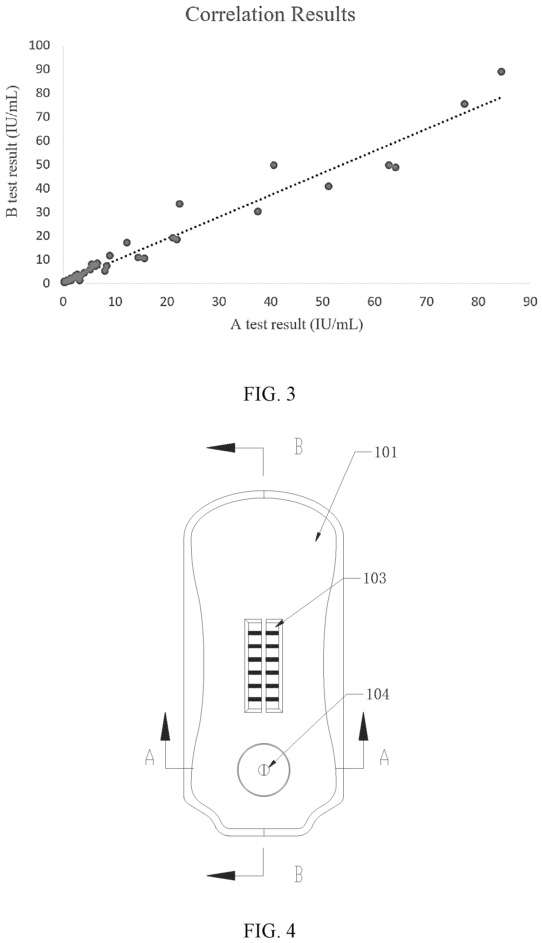

Since the Phadia test kit is recognized as the gold standard for the testing of allergen-specific IgE antibodies, comparative testing was performed using both the Phadia test kit and the multiplex quantum dot-based quantitative test card to simultaneously analyze samples containing specific IgE antibodies against at least any one of the inhaled or ingested allergens described in the present application. The corresponding test results are shown in Table 2 and FIG. 3 (where the A test result represents the test result obtained from the Phadia test kit, and the B test result represents the test result obtained from the multiplex quantum dot-based quantitative test card). As shown in FIG. 3, the correlation between the test results obtained from the Phadia test kit and from the multiplex quantum dot-based quantitative test card is represented by the formula y = 0.9244*x*+ 0.2388, where R² = 0.957. FIG. 3 demonstrates a good correlation between the test results obtained from the present application and from the Phadia test kit as the industry-recognized gold standard, thereby proving that the multiplex quantum dot-based quantitative test card of the present application can provide test results with high accuracy.

**Table 2 Correlation Test Results of Levels of Allergen-specific IgE Antibodies in Serum Samples (IU/mL)**

| | A test result | B test result | | A test result | B test result |
|---|---|---|---|---|---|
| Result 1 | 0.33 | 0.23 | Result 21 | 6.62 | 8.22 |
| Result 2 | 1.23 | 1.01 | Result 22 | 51.2 | 40.6 |
| Result 3 | 0.35 | 0.52 | Result 23 | 5.24 | 5.29 |
| Result 4 | 1.68 | 1.45 | Result 24 | 1.52 | 1.77 |
| Result 5 | 8.57 | 7.21 | Result 25 | 14.6 | 10.5 |
| Result 6 | 77.5 | 75.4 | Result 26 | 6.24 | 6.91 |
| Result 7 | 4.22 | 4.17 | Result 27 | 3.32 | 1.25 |
| Result 8 | 1.57 | 1.02 | Result 28 | 62.8 | 49.5 |
| Result 9 | 2.93 | 3.35 | Result 29 | 3.45 | 2.93 |
| Result 10 | 9.1 | 11.4 | Result 30 | 21.2 | 18.9 |
| Result 11 | 5.52 | 7.89 | Result 31 | 0.51 | 0.44 |
| Result 12 | 37.6 | 29.9 | Result 32 | 0.45 | 0.27 |
| Result 13 | 2.47 | 2.98 | Result 33 | 0.38 | 0.31 |
| Result 14 | 2.88 | 3.41 | Result 34 | 1.92 | 1.67 |
| Result 15 | 84.6 | 88.9 | Result 35 | 8.11 | 5.12 |
| Result 16 | 12.4 | 16.8 | Result 36 | 64.2 | 48.7 |
| Result 17 | 22.5 | 33.4 | Result 37 | 0.78 | 1.01 |
| Result 18 | 21.9 | 18.3 | Result 38 | 0.99 | 0.83 |
| Result 19 | 1.42 | 1.28 | Result 39 | 15.7 | 10.3 |
| Result 20 | 2.56 | 2.15 | Result 40 | 40.7 | 49.3 |

### Example II

This example provides a preparation method of a multiplex quantum dot-based quantitative test card, where the preparation method includes the following steps:
S1: preparing a coated nitrocellulose membrane containing a plurality of test lines and a control line.

Specifically, the step of preparing the coated nitrocellulose membrane containing the plurality of test lines and the control line includes:
fixedly adhering a nitrocellulose membrane to a central portion of a backing plate; and
immobilizing allergen antigens and antibodies diluted to desired coating concentrations onto the nitrocellulose membrane, and performing drying to obtain the coated nitrocellulose membrane.

Herein, the allergen antigens include any one selected from the group consisting of: cat dander, mugwort, birch trees, timothy grass, cockroaches, dog dander, plantains, ragweed, house dust mites, *Alternaria alternata* (inhaled allergens), peanuts, milk, codfish, scallops, chicken egg white, shrimp, crabs, soybeans, wheat, and almonds (ingested allergens); the antibodies are goat anti-chicken IgY antibodies.

Preferably, the desired coating concentration of the allergen antigens is 0.5-2 mg/mL. For example, the coating concentration of the allergen antigens may be 0.5 mg/mL, 1 mg/mL, 1.5 mg/mL, or 2 mg/mL. Those skilled in the art may make adjustments as needed, which will not be elaborated herein.

Preferably, the desired coating concentration of the goat anti-chicken IgY antibodies is 1-3 mg/mL. For example, the coating concentration of the goat anti-chicken IgY antibodies may be 1 mg/mL, 1.5 mg/mL, 2 mg/mL, 2.5 mg/mL, or 3 mg/mL. Those skilled in the art may make adjustments as needed, which will not be elaborated herein.

Preferably, the nitrocellulose membrane coated with the allergen antigens and the antibodies is dried at 37°C overnight.

S2: preparing a conjugate pad coated with two types of quantum dot-labeled antibodies, where the two types of quantum dot-labeled antibodies include: quantum dot-labeled mouse anti-human IgE antibodies and quantum dot-labeled chicken IgY antibodies.

Specifically, the step of preparing the conjugate pad coated with the two types of quantum dot-labeled antibodies includes:
obtaining a quantum dot solution, where
quantum dots are added to a 4-Morpholineethanesulfonic acid buffer at pH 6, followed by sequential addition of 1-(3-Dimethylaminopropyl)-3-ethylcarbodiimide hydro (EDC) and N-Hydroxysuccinimide (NHS) at a certain mass ratio; after rotary mixing for 30 min to complete activation, the supernatant is removed by centrifugation, and the quantum dot solids are resuspended in either the 4-Morpholineethanesulfonic acid buffer at pH 6 or a borate buffer at pH 8 via agitation to obtain the quantum dot solution.

Preferably, the EDC and the NHS are added at a mass ratio of 1:2.
preparing the quantum dot-labeled mouse anti-human IgE antibodies and the quantum dot-labeled chicken IgY antibodies, where
mouse anti-human IgE antibodies or chicken IgY antibodies are added to the quantum dot solution, followed by rotary mixing for 2 h to facilitate conjugation; a blocking solution is then added for rotary blocking for additional 30 min; after removing the supernatant, the quantum dot solids are resuspended in a conjugate pad treatment buffer to obtain the quantum dot-labeled mouse anti-human IgE antibodies or the quantum dot-labeled chicken IgY antibodies.

Preferably, the concentration of the quantum dot-labeled mouse anti-human IgE antibodies or the quantum dot-labeled chicken IgY antibodies is 20-300 µg/mL. For example, the concentration of the quantum dot-labeled mouse anti-human IgE antibodies or the quantum dot-labeled chicken IgY antibodies may be 20 µg/mL, 70 µg/mL, 110 µg/mL, 150 µg/mL, 180 µg/mL, 220 µg/mL, 260 µg/mL, or 300 µg/mL. Those skilled in the art may make adjustments as needed, which will not be elaborated herein.

Preferably, the blocking solution is a mixed solution containing at least one selected from the group consisting of: 1-10% casein, 1-10% polyvinylpyrrolidone K30 (PVP-30), 1-10% mouse IgG antibodies, and 1-10% glycine. The blocking solution is conducive to mitigating cross-reactivity between test items and nonspecific interference from the test samples.

Herein, the conjugate pad treatment solution contains a saccharide, a macromolecular polymer, a surfactant, a diluent, a protein, and a preservative.

Preferably, the saccharide includes at least one selected from the group consisting of: trehalose, sucrose, and dextran.

Preferably, the macromolecular polymer includes at least one selected from the group consisting of: polyethylene glycol-20000 (PEG-20000), PVP-30, polyvinylpyrrolidone K10 (PVP-10), and polyethylene glycol-8000 (PEG-8000).

Preferably, the surfactant includes at least one selected from the group consisting of: Triton X-100 (TX-100), Tween-20 (TW-20), and Pluracare 1307 (S9, a propylene oxide-ethylene oxide-ethylenediamine copolymer).

Preferably, the diluent includes at least one selected from the group consisting of: phosphate buffered saline (PBS), tris(hydroxymethyl)aminomethane (Tris), and borate buffered saline (BBS).

Preferably, the protein includes at least one selected from the group consisting of: casein, bovine serum albumin, and skim milk.

Preferably, the preservative includes at least one selected from the group consisting of: sodium azide (NaN₃) and ProClin300 (PC-300).

Herein, the macromolecular polymer is conducive to enhancing antibody activity; the surfactant is conducive to facilitating chromatographic flow of liquids; the protein is conducive to optimizing liquid chromatography and reducing nonspecific binding in test samples, and the preservative is conducive to improving stability of the conjugate pad.

The quantum dot-labeled mouse anti-human IgE antibodies and the quantum dot-labeled chicken IgY antibodies are mixed in proportion; a resulting mixture is then immobilized onto the conjugate pad; and after drying, the conjugate pad coated with the two types of quantum dot-labeled antibodies is obtained.

Preferably, the quantum dot-labeled mouse anti-human IgE antibodies and the quantum dot-labeled chicken IgY antibodies are mixed at a ratio ranging from 3:1 to 10:1. For example, the quantum dot-labeled mouse anti-human IgE antibodies and the quantum dot-labeled chicken IgY antibodies may be mixed at a ratio of 3:1, 5:1, 8:1, or 10:1. Those skilled in the art may make adjustments as needed, which will not be elaborated herein.

Preferably, the conjugate pad coated with the two types of quantum dot-labeled antibodies is dried at 37°C overnight.

S3: assembling the coated nitrocellulose membrane and the conjugate pad coated with the two types of quantum dot-labeled antibodies to obtain the multiplex quantum dot-based quantitative test card.

Specifically, the step of assembling the coated nitrocellulose membrane and the conjugate pad coated with the two types of quantum dot-labeled antibodies includes:
assembling the coated nitrocellulose membrane, the conjugate pad coated with the two types of quantum dot-labeled antibodies, a sample pad, an absorbent pad, and the backing plate to obtain a test strip, where
the absorbent pad is fixedly adhered to an edge, proximal to the control line, of the coated nitrocellulose membrane, and the conjugate pad coated with the two types of quantum dot-labeled antibodies is fixedly adhered to an edge, proximal to the test lines, of the coated nitrocellulose membrane; the sample pad is fixedly adhered to an edge, distal to the test lines, of the conjugate pad coated with the two types of quantum dot-labeled antibodies; and the backing plate with the coated nitrocellulose membrane, the absorbent pad, the conjugate pad coated with the two types of quantum dot-labeled antibodies, and the sample pad fixedly adhered thereto is cut into a strip of 3 mm to obtain the test strip.

The test strip is fixedly positioned within an accommodation slot of a multiplex card housing to obtain the multiplex quantum dot-based quantitative test card.

### Example III

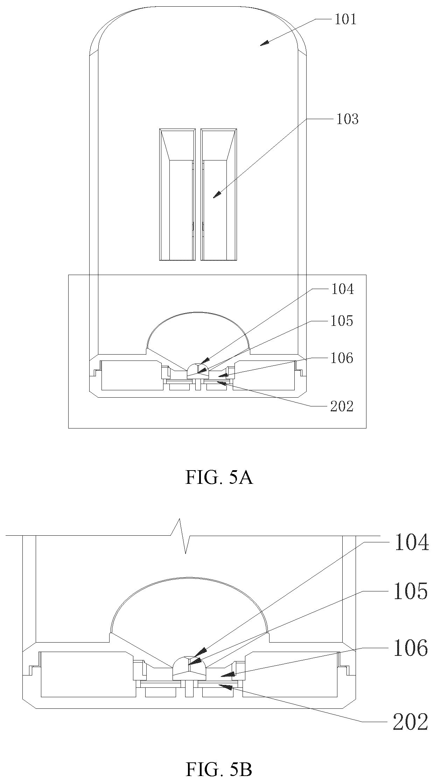

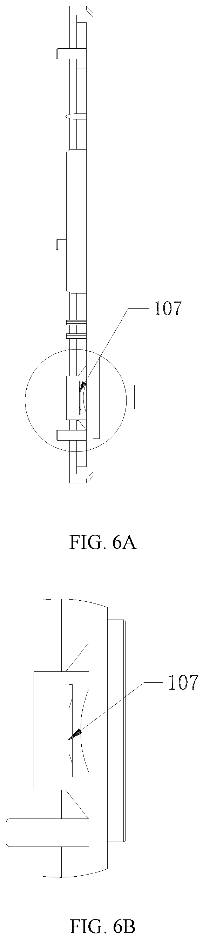

FIGs. 4-8 show a multiplex testing apparatus provided in this example, including a test card 100 and a test strip 200 adapted for use with the test card.

FIGs. 4-7 show the test card of the multiplex testing apparatus provided in this example.

The test card 100 includes a first housing 101 and a second housing 102 (not shown), where the first housing 101 is provided with a sample loading port 104 configured for single-loading of a test sample.

The first housing 101 further includes a flow dividing chamber disposed at a position where the sample loading port 104 is located, where the flow dividing chamber includes a flow divider 105 configured to bifurcate the test sample loaded through the sample loading port 104 into two flow paths.

The flow divider 105 is positioned in center alignment with the sample loading port 104; and the flow dividing chamber includes two outlet orifices 106 respectively disposed at two sides of the flow divider 105, where following test sample bifurcation by the flow divider 105, the two test sample portions can be independently discharged through the respective outlet orifices 106.

Meanwhile, the second housing 102 is provided with two accommodation slots (not marked) configured to accommodate the test strip 100; when the first housing 101 and the second housing 102 are connected, the two outlet orifices 106 are respectively in fluid communication with the two accommodation slots, thereby enabling direct transfer of the bifurcated test sample portions into the accommodation slots for immediate testing by the test strip. This configuration prevents test sample loss while reducing the required test sample volume.

Herein, the first housing 101 further includes a buffer zone (not marked) around the sample loading port 104. Since the flow of the test sample and the sample diluent requires a certain amount of time, the buffer zone is configured to temporarily retain the test sample loaded through the sample loading port and the sample diluent.

Preferably, the buffer zone and the sample loading port 104 collectively form a circular truncated cone structure, where the sample loading port 104 constitutes the smaller-diameter circular surface of the structure; the sample loading port has a diameter of 11.6 mm; and the outlet orifices each have a diameter of 2.46 mm.

Preferably, the flow divider is a wedge-shaped component including two inclined surfaces arranged symmetrically about the center of the flow divider in their inclination directions, where the angle between each inclined surface and the vertical direction is preferably 110°.

Preferably, the flow divider is made of a plastic material selected from the group consisting of: acrylonitrile butadiene styrene (ABS), polystyrene (PS), high impact polystyrene (HIPS), and polycarbonate (PC).

Since the test sample is a blood sample (including a whole blood, plasma, or serum sample), which exhibits higher relative viscosity compared to water, the test sample tends to resist downward flow when loaded through the sample loading port. To enhance the fluidity of the test sample, in this example, vent holes 107 are formed in both side walls of the first housing 101 and positioned respectively in correspondence with the two outlet orifices 106. Therefore, trapped air within the outlet orifices 106 escapes through the vent holes 107 during test sample loading, thereby ensuring unimpeded test sample flow and preventing clogging of the sample loading port 104.

Meanwhile, the first housing 101 is provided with two observation windows 103, where the two observation windows 103 are arranged respectively in one-to-one correspondence with the two accommodation slots to facilitate a user's observation of the test strip.

The assembled test card 100 is sealed and stored in an aluminum foil pouch containing a desiccant.

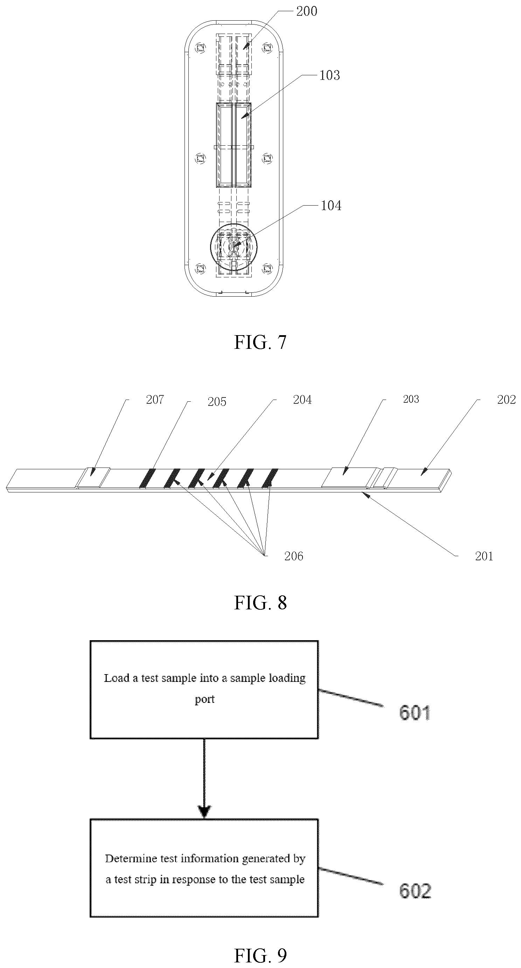

FIG. 8 shows the test strip of the multiplex testing apparatus provided in this example.

The test strip 200 includes a first end and a second end, where when the test strip 200 is positioned within the accommodation slots during use, the first end is positioned in correspondence with the outlet orifices 106.

The test strip 200 includes a backing plate 201, and a sample pad 202, a colloidal gold pad 203, a nitrocellulose membrane 204, and an absorbent pad 207 sequentially disposed on the backing plate 201 along a direction extending from the first end to the second end, where the sample pad 202 is positioned in correspondence with the outlet orifices 106 so that the test sample discharged from the outlet orifices 106 can be directly absorbed by the sample pad 202, thereby reducing test sample loss.

It should be noted that the first and second ends mentioned herein are defined according to the flow direction of the test sample. During operation of the test strip, the test sample migrates through chromatography from the sample pad end (positioned at the first end) toward the absorbent pad end (positioned at the second end).

Preferably, the sample pad 202 is a polyester fiber membrane or a glass fiber membrane.

Preferably, the colloidal gold pad 203 is a glass fiber membrane.

Preferably, the nitrocellulose membrane 204 is coated with a plurality of test lines 206 and a control line 205. During use, the test lines are configured to indicate test results generated by the multiplex testing apparatus, while the control line is configured to indicate validity of the test results. In this example, the test lines are provided in a total of five, where the five test lines are uniformly spaced along a lengthwise direction of the test strip proximal to the first end, while the control line is arranged proximal to the second end.

Herein, the nitrocellulose membrane is positioned in correspondence with the observation windows to facilitate a user's observation of the test results generated by the testing apparatus.

Herein, the five test lines are coated with multiple types of allergen antigens, while the control line is coated with goat anti-chicken IgY antibodies. The allergen antigens coated on the test lines can bind to -colloidal gold particle-labeled mouse anti-human IgE antibodies to produce a color signal, enabling visual observation of test results; the goat anti-chicken IgY antibodies coated on the control line can bind to colloidal gold particle-labeled mouse anti-human IgE antibodies and colloidal gold particle-labeled chicken IgY antibodies respectively, both producing a red signal to indicate proper testing by the test strip.

Herein, each of the test lines is coated with a distinct one of the multiple types of allergen antigens.

Preferably, the multiple types of allergen antigens include inhaled allergen antigens and ingested allergen antigens.

Preferably, the inhaled allergen antigens include one selected from the group consisting of: cat dander, mugwort, birch trees, timothy grass, cockroaches, dog dander, plantains, ragweed, house dust mites, and *Alternaria alternata.*

Preferably, the ingested allergen antigens include one selected from the group consisting of: peanuts, milk, codfish, scallops, chicken egg white, shrimp, crabs, soybeans, wheat, and almonds.

For example, the plurality of test lines include a first test line, a second test line, a third test line, a fourth test line, and a fifth test line, where the first test line is coated with a crude extract antigen of cat dander; the second test line is coated with a crude extract antigen of peanuts; the third test line is coated with a crude extract antigen of birch trees; the fourth test line is coated with a crude extract antigen of chicken egg white; and the fifth test line is coated with a crude extract antigen of cockroaches. Those skilled in the art may make adjustments as needed, which will not be elaborated herein.

Preferably, the desired coating concentration of the allergen antigens is 0.1-3 mg/mL. For example, the coating concentration of the allergen antigens may be 0.1 mg/mL, 0.2 mg/mL, 0.5 mg/mL, 1 mg/mL, 1.5 mg/mL, 2 mg/mL, 2.5 mg/mL, or 3 mg/mL. Those skilled in the art may make adjustments as needed, which will not be elaborated herein.

Preferably, the desired coating concentration of the goat anti-chicken IgY antibodies is 1-3 mg/mL. For example, the coating concentration of the goat anti-chicken IgY antibodies may be 1 mg/mL, 1.5 mg/mL, 2 mg/mL, 2.5 mg/mL, or 3 mg/mL. Those skilled in the art may make adjustments as needed, which will not be elaborated herein.

Preferably, antibodies coated on the colloidal gold pad are colloidal gold particle-labeled mouse anti-human IgE antibodies and colloidal gold particle-labeled chicken IgY antibodies.

Preferably, the colloidal gold particle-labeled mouse anti-human IgE antibodies and the colloidal gold particle-labeled chicken IgY antibodies coated on the colloidal gold pad are mixed at a ratio ranging from 10:1 to 3:1. For example, the colloidal gold particle-labeled mouse anti-human IgE antibodies and the colloidal gold particle-labeled chicken IgY antibodies may be mixed at a ratio of 10:1, 8:1, 5:1, or 3:1. Those skilled in the art may make adjustments as needed, which will not be elaborated herein.

Preferably, the concentrations of the colloidal gold particle-labeled mouse anti-human IgE antibodies and the colloidal gold particle-labeled chicken IgY antibodies coated on the colloidal gold pad are 5-25 µg/mL. For example, the concentrations of the colloidal gold particle-labeled mouse anti-human IgE antibodies and the colloidal gold particle-labeled chicken IgY antibodies may be 5 µg/mL, 10 µg/mL, 15 µg/mL, or 25 µg/mL. Those skilled in the art may make adjustments as needed, which will not be elaborated herein.

Specifically, the method for preparing the test strip may include: adhering the coated nitrocellulose membrane 204 to a central portion of the backing plate 201; adhering one end of the absorbent pad 207 to the second end of the backing plate while overlapping the other end of the absorbent pad 207 onto the nitrocellulose membrane 204; overlapping one end of the colloidal gold pad 203 onto the nitrocellulose membrane 204 proximal to the test lines 206 while adhering the other end of the colloidal gold pad to the backing plate 201; adhering one end of the sample pad 202 to the first end of the backing plate 201 while overlapping the other end of the sample pad onto the colloidal gold pad 203; and cutting the assembled structure into a strip of 3 mm in width using a cutter to obtain the test strip 200 of the multiplex testing apparatus of the present application.

This example further discloses a multiplex testing apparatus, including: in addition to the test card and the test strip, a sample diluent, where during use, the sample diluent is used in conjunction with a test sample and added after the test sample is loaded. The sample diluent is conducive to preventing premature coagulation of the test sample, thereby avoiding testing failure, and also to diluting the concentration of the test sample, thereby improving testing accuracy.

Herein, the sample diluent contains a buffer solution, a macromolecular polymer, a surfactant, and a preservative, which collectively help to stabilize the tertiary structures of antibodies and enhance chromatographic performance of blood samples on the nitrocellulose membrane.

Preferably, the buffer solution includes at least one selected from the group consisting of: phosphate buffered saline (PBS), tris(hydroxymethyl)aminomethane (Tris), and borate buffered saline (BBS); the macromolecular polymer includes at least one selected from the group consisting of: polyethylene glycol (PEG), PVP-30, polyvinyl pyrrolidone (PVP), and poly(vinyl alcohol)(PVA); the surfactant includes at least one selected from the group consisting of: Triton and Tween; and the preservative includes at least one selected from the group consisting of: sodium azide (NaN₃) and ProClin300 (PC-300). This example further discloses components of a sample pad treatment solution in the test strip.

The sample pad treatment solution contains a buffer solution, a macromolecular polymer, a surfactant, a preservative, a blocker, and a whole blood sample treatment additive, which collectively help to filter particulate matters, enhance specific reactions, reduce interference from endogenous substances, and optimize chromatographic performance on the nitrocellulose membrane when whole blood samples are processed.

Preferably, the blocker includes at least one selected from the group consisting of: human anti-animal antibody (e.g., human anti-mouse antibody) blockers, rheumatoid factor blockers, heterophilic antibody blockers, complement blockers, and autoantibody blockers; and the whole blood sample treatment additive includes at least one selected from the group consisting of: anti-erythrocyte antibodies and phytohemagglutinin. This example further discloses components of a colloidal gold pad treatment solution in the test strip, including a buffer solution, a macromolecular polymer, a surfactant, a preservative, and a saccharide, which collectively help to improve antibody sensitivity and antibody stability in a solid phase.

Preferably, the saccharide includes at least one selected from the group consisting of: trehalose, sucrose, and dextran.

Preferably, in the sample diluent, sample pad treatment solution, and colloidal gold pad treatment solution, the concentrations of the buffer, macromolecular polymer, surfactant, preservative, whole blood sample treatment additive, and saccharide are 10-200 mM, 0.1-5%, 0.1-3%, 0.01-2%, 10-50 µg/mL, and 0.5-5% respectively.

Following optimized treatment of the sample pad and colloidal gold pad of the test strip in the testing apparatus using the sample pad treatment solution and colloidal gold pad treatment solution, the testing apparatus demonstrates improved positive percent agreement (PPA) and negative percent agreement (NPA) for allergen testing. The test results of various coated inhaled and ingested allergens using the multiplex testing apparatuses non-optimized and optimized with the conjugate pad treatment solution are shown in Tables 3 and 4, respectively. The test results show that both the multiplex testing apparatuses meet industry standards for PPA/NPA, and the optimized testing apparatus exhibits superior PPA/NPA performance relative to the non-optimized testing apparatus.

**Table 3 Serum Sample Test Results (1)**

| | Non-optimized with the conjugate pad treatment solution | | Optimized with the conjugate pad treatment solution | |
|---|---|---|---|---|
| Test item | PPA | NPA | PPA | NPA |
| Cat dander | 11/17 | 14/20 | 14/17 | 17/20 |
| Mugwort | 10/15 | 15/20 | 13/15 | 18/20 |
| Birch tree | 9/19 | 14/20 | 14/19 | 17/20 |
| Timothy grass | 8/13 | 15/20 | 10/13 | 17/20 |
| Cockroach | 11/15 | 15/20 | 11/15 | 18/20 |
| Dog dander | 10/16 | 17/20 | 12/16 | 18/20 |
| Plantain | 6/10 | 13/20 | 8/10 | 16/20 |
| Ragweed | 9/14 | 14/20 | 11/14 | 17/20 |
| House dust mite | 9/16 | 16/20 | 11/16 | 17/20 |
| *Alternaria alternata* | 10/16 | 15/20 | 13/16 | 18/20 |
| Peanut | 8/13 | 13/20 | 11/13 | 15/20 |
| Milk | 9/15 | 12/20 | 11/15 | 14/20 |
| Codfish | 12/19 | 13/20 | 15/19 | 15/20 |
| Scallop | 11/17 | 14/20 | 13/17 | 16/20 |
| Chicken egg white | 10/18 | 15/20 | 13/18 | 16/20 |
| Shrimp | 11/16 | 16/20 | 13/16 | 18/20 |
| Crab | 8/13 | 14/20 | 11/13 | 16/20 |
| Soybean | 11/16 | 14/20 | 13/16 | 15/20 |
| Wheat | 7/10 | 13/20 | 8/10 | 16/20 |
| Almond | 9/14 | 15/20 | 12/14 | 17/20 |

**Table 4 Serum Sample Test Results (2)**

| | Non-optimized with the sample pad treatment solution | | Optimized with the sample pad treatment solution | |
|---|---|---|---|---|
| Test item | PPA | NPA | PPA | NPA |
| Cat dander | 11/17 | 14/20 | 12/17 | 18/20 |
| Mugwort | 10/15 | 15/20 | 12/15 | 17/20 |
| Birch tree | 9/19 | 14/20 | 15/19 | 18/20 |
| Timothy grass | 8/13 | 15/20 | 11/13 | 18/20 |
| Cockroach | 11/15 | 15/20 | 13/15 | 17/20 |
| Dog dander | 10/16 | 17/20 | 13/16 | 17/20 |
| Plantain | 6/10 | 13/20 | 9/10 | 17/20 |
| Ragweed | 9/14 | 14/20 | 12/14 | 16/20 |
| House dust mite | 9/16 | 16/20 | 12/16 | 17/20 |
| *Alternaria alternata* | 10/16 | 15/20 | 13/16 | 18/20 |
| Peanut | 8/13 | 13/20 | 11/13 | 15/20 |
| Milk | 9/15 | 12/20 | 12/15 | 16/20 |
| Codfish | 12/19 | 13/20 | 14/19 | 16/20 |
| Scallop | 11/17 | 14/20 | 13/17 | 15/20 |
| Chicken egg white | 10/18 | 15/20 | 14/18 | 17/20 |
| Shrimp | 11/16 | 16/20 | 14/16 | 17/20 |
| Crab | 8/13 | 14/20 | 10/13 | 17/20 |
| Soybean | 11/16 | 14/20 | 14/16 | 15/20 |
| Wheat | 7/10 | 13/20 | 8/10 | 17/20 |
| Almond | 9/14 | 15/20 | 11/14 | 18/20 |

In the aforementioned sample diluent, sample pad treatment solution, and colloidal gold pad treatment solution, the macromolecular polymer can enhance antibody activity and maintain proper spatial conformation of antibodies; the blocker can improve specific reactions while mitigating interference from endogenous substances and non-target protein antibodies in test samples; the whole blood sample treatment additive can facilitate filtration of particulate matters in test samples, enabling unimpeded sample flow through the nitrocellulose membrane; and the preservative can ensure stability of both the sample pad and the colloidal gold pad. Therefore, the combination of sample diluent-treated test samples with optimized test strips can enhance both the PPA and NPA of the testing apparatus.

### Example IV

FIG. 9 is a flow chart of a use method of a multiplex testing apparatus.

Referring to FIG. 9, this example provides a use method of a multiplex testing apparatus, where the use method includes the following steps:
S601: loading a test sample into a sample loading port.

Specifically, the test sample is a blood sample, which may be a whole blood sample, a plasma sample, or a serum sample.

Specifically, detectable allergens include: cat dander, mugwort, birch trees, timothy grass, cockroaches, dog dander, plantains, ragweed, house dust mites, *Alternaria alternata,* peanuts, milk, codfish, scallops, chicken egg white, shrimp, crabs, soybeans, wheat, and almonds.

S602: determining test information generated by a test strip in response to the test sample.

Specifically, upon sequential addition of a test sample of 60 µL followed by a sample diluent of 60 µL through the sample loading port, the test sample and the sample diluent are bifurcated by a flow divider and directed through two outlet orifices to sample pads of two test strips respectively in a test card.

Furthermore, the flow divider bifurcates the test sample and the sample diluent into two equal portions, with each portion being directed through the flow divider's two inclined surfaces toward respective outlet orifices.

Preferably, the two test strips disposed in a second housing are physically isolated from each other, enabling simultaneous immunochromatographic reactions to proceed independently in both the two test strips upon loading of the test sample and the sample diluent.

Preferably, the testing apparatus is capable of simultaneously testing ten distinct allergens.

The testing apparatus of the present application requires only single-port/single-sample-loading and eliminates the need for repeated sample collection or multiple loadings while achieving simultaneous testing of ten distinct allergens in one operation, thereby offering exceptional operational simplicity and clinical utility.

The present application employs highly specific IgE antibody-antigen interactions, where the colloidal gold particle-labeled mouse anti-human IgE antibodies and the colloidal gold particle-labeled chicken IgY antibodies are immobilized on the colloidal gold pad. Utilizing immunochromatographic technology, the test sample, after dilution with the sample diluent, migrates to combine with the colloidal gold particle-labeled mouse anti-human IgE antibodies coated on the colloidal gold pad to form a labeled complex that subsequently binds to antigens coated on the test lines, generating visual red or pink spots. The goat anti-chicken IgY antibodies coated on the control line bind to the remaining colloidal gold particle-labeled mouse anti-human IgE antibodies and colloidal gold particle-labeled chicken IgY antibodies, generating visual red or pink spots. Through ligand-directed accumulation of colloidal gold particle-labeled antibodies, color development occurs at allergen-coated test lines on the nitrocellulose membrane, enabling visual observation of test results generated by the multiplex testing apparatus via the observation windows.

In the multiplex testing apparatus of the present application, the test strip includes a plurality of test lines. Following the binding of the test sample with colloidal gold particle-labeled mouse anti-human IgE antibodies and subsequent interaction with allergen antigens coated on the test lines, two potential scenarios may occur: 1) sufficient remaining colloidal gold particle-labeled mouse anti-human IgE antibodies migrate to the control line, resulting in color development; or 2) insufficient or no colloidal gold particle-labeled mouse anti-human IgE antibodies migrate to the control line, resulting in no color development. Consequently, to prevent the occurrence of the second scenario described above, the present application coats both colloidal gold particle-labeled mouse anti-human IgE antibodies and colloidal gold particle-labeled chicken IgY antibodies on the colloidal gold pad. Even if the second scenario described above occurs, the goat anti-chicken IgY antibodies coated on the control line will still bind to the colloidal gold particle-labeled chicken IgY antibodies to enable color development, thereby ensuring the validity of test results (whether negative or positive) generated by the testing apparatus. Therefore, the multiplex testing apparatus of the present application not only enables simultaneous specific testing of multiple types of distinct allergen antigens but also prevents invalid test results from the test strip, thereby reducing the need for repeated testing.

The test results from the test strip are interpreted as follows:
positive: a red band appears on both the control line and a test line, indicating the presence of IgE antibodies in the test sample specific to the corresponding allergen coated on the test line;
negative: a red band appears on the control line with no red band on any test line, indicating the absence of IgE antibodies in the test sample specific to the corresponding allergen coated on the test line; and
invalid: no red band appears on the control line, regardless of a red band on any test line, indicating the test result is invalid.

The multiplex testing apparatus and the use method thereof provided by the present application are described above in detail. The principle and implementation manners of the present application are elaborated by applying specific examples. The descriptions of the above embodiments are only used to help understand the method and its core idea of the present application; meanwhile, according to the idea of the present application, there will be changes in the implementation manners and the application scope for a person of ordinary skill in the art. In summary, the content of the specification should not be understood as limiting the present application.

It should be noted that the orientations or positional relationships indicated by the terms "central", "longitudinal", "transverse", "up", "down", "front", "back", "left", "right", "vertical", "horizontal", "top", "bottom", "inside", "outside", and the like as used herein are based on those shown in the accompanying drawings, intended only for the convenience of describing the present application and simplifying the description rather than for indicating or implying that the referred apparatus or element must be provided with a particular orientation or constructed and operated with a particular orientation, and consequently not allowed to be construed as limiting the present application. Furthermore, the terms "first" and "second" are intended only for descriptive purposes and should not be construed as indicating or implying their relative importance or implicitly indicating the quantity of technical features indicated. Therefore, features defined by "first" and "second" may explicitly or implicitly include one or more of such features. In the description of the present application, unless otherwise specified, "a plurality of' refers to two or more.

The above description is only the preferred embodiments of the present application and the description of the applied technical principles. A person skilled in the art should understand that the scope of the invention involved in the present application is not limited to the technical solutions formed by a specific combination of the described technical features, and should also cover other technical solutions formed by any combination of the described technical features or equivalent features thereof without departing from the concept of the invention. For example, the technical solution formed by replacing the described features with technical features having similar functions disclosed in the present application (but not limited thereto).

## Claims

1. A multiplex quantum dot-based quantitative test card, wherein the test card comprises:
a multiplex card housing and a test strip, wherein
the multiplex card housing is provided with a plurality of reaction slots configured to accommodate the test strip;
the multiplex card housing further comprises a plurality of sample loading ports and a plurality of observation windows, wherein the sample loading ports and the observation windows are in one-to-one correspondence with the reaction slots; and
the test strip comprises a conjugate pad coated with quantum dot-labeled antibodies, wherein
the quantum dot-labeled antibodies serve as chromogenic substances.

2. The multiplex quantum dot-based quantitative test card of claim 1, wherein the test strip further comprises a sample pad, a nitrocellulose membrane, an absorbent pad, and a backing plate, wherein
the sample pad, the conjugate pad, the nitrocellulose membrane, and the absorbent pad are sequentially and uniformly adhered to the backing plate; and
the nitrocellulose membrane is coated with a plurality of test lines and a control line, wherein the plurality of test lines and the control line are uniformly spaced and immobilized on the nitrocellulose membrane.

3. The multiplex quantum dot-based quantitative test card of claim 2, wherein the plurality of test lines are coated with distinct allergen antigens, while the control line is coated with goat anti-chicken IgY antibodies.

4. The multiplex quantum dot-based quantitative test card of claim 3, wherein the allergen antigens comprise at least any one selected from the group consisting of: cat dander, mugwort, birch trees, timothy grass, cockroaches, dog dander, plantains, ragweed, house dust mites, *Alternaria alternata* (inhaled allergens), peanuts, milk, codfish, scallops, chicken egg white, shrimp, crabs, soybeans, wheat, and almonds (ingested allergens).

5. The multiplex quantum dot-based quantitative test card of claim 1, wherein the quantum dot-labeled antibodies coated on the conjugate pad are mouse anti-human IgE antibodies and chicken IgY antibodies.

6. A preparation method of a multiplex quantum dot-based quantitative test card, used for preparing the multiplex quantum dot-based quantitative test card of any one of claims 1-5, wherein the preparation method comprises the following steps:
preparing a coated nitrocellulose membrane containing a plurality of test lines and a control line;
preparing a conjugate pad coated with two types of quantum dot-labeled antibodies, wherein the two types of quantum dot-labeled antibodies comprise: quantum dot-labeled mouse anti-human IgE antibodies and quantum dot-labeled chicken IgY antibodies; and
assembling the coated nitrocellulose membrane and the conjugate pad coated with the two types of quantum dot-labeled antibodies to obtain the multiplex quantum dot-based quantitative test card.

7. The preparation method of the multiplex quantum dot-based quantitative test card of claim 6, wherein the step of preparing the coated nitrocellulose membrane containing the plurality of test lines and the control line comprises;
fixedly adhering a nitrocellulose membrane to a central portion of a backing plate; and
immobilizing allergen antigens and antibodies diluted to desired coating concentrations onto the nitrocellulose membrane, and performing drying to obtain the coated nitrocellulose membrane.

8. The preparation method of the multiplex quantum dot-based quantitative test card of claim 6, wherein the step of preparing the conjugate pad coated with the two types of quantum dot-labeled antibodies comprises:
obtaining a quantum dot solution;
preparing the quantum dot-labeled mouse anti-human IgE antibodies and the quantum dot-labeled chicken IgY antibodies; and
mixing the quantum dot-labeled mouse anti-human IgE antibodies and the quantum dot-labeled chicken IgY antibodies in proportion, immobilizing a resulting mixture onto the conjugate pad, and performing drying to obtain the conjugate pad coated with the two types of quantum dot-labeled antibodies.

9. The preparation method of the multiplex quantum dot-based quantitative test card of claim 6, wherein the step of assembling the coated nitrocellulose membrane and the conjugate pad coated with the two types of quantum dot-labeled antibodies comprises:
assembling the coated nitrocellulose membrane, the conjugate pad coated with the two types of quantum dot-labeled antibodies, a sample pad, an absorbent pad, and the backing plate to obtain a test strip; and
fixedly positioning the test strip within an accommodation slot of a multiplex card housing to obtain the multiplex quantum dot-based quantitative test card.

10. The preparation method of the multiplex quantum dot-based quantitative test card of claim 9, wherein the step of assembling the coated nitrocellulose membrane, the conjugate pad coated with the two types of quantum dot-labeled antibodies, the sample pad, the absorbent pad, and the backing plate to obtain the test strip comprises:
fixedly adhering the absorbent pad to an edge, proximal to the control line, of the coated nitrocellulose membrane, and fixedly adhering the conjugate pad coated with the two types of quantum dot-labeled antibodies to an edge, proximal to the test lines, of the coated nitrocellulose membrane;
fixedly adhering the sample pad to an edge, distal to the test lines, of the conjugate pad coated with the two types of quantum dot-labeled antibodies; and
cutting the backing plate with the coated nitrocellulose membrane, the absorbent pad, the conjugate pad coated with the two types of quantum dot-labeled antibodies, and the sample pad fixedly adhered thereto into a strip of 2-6 mm to obtain the test strip.

11. A multiplex testing apparatus, comprising:
a test card and a test strip adapted for use with the test card, wherein
the test card comprises a first housing and a second housing, wherein the second housing is provided with two accommodation slots configured to accommodate the test strip;
the first housing is provided with a sample loading port and a flow dividing chamber disposed at a position where the sample loading port is located, wherein the flow dividing chamber comprises a flow divider positioned in center alignment with the sample loading port; and
the flow dividing chamber comprises two outlet orifices respectively disposed at two sides of the flow divider, wherein the two outlet orifices are in fluid communication with the two accommodation slots.

12. The multiplex testing apparatus of claim 11, wherein vent holes are formed in both side walls of the first housing and positioned respectively in correspondence with the two outlet orifices.

13. The multiplex testing apparatus of claim 11, wherein the first housing is provided with two observation windows; and
the two observation windows are arranged respectively in one-to-one correspondence with the two accommodation slots.

14. The multiplex testing apparatus of claim 11, wherein the test strip comprises a first end and a second end, and when the test strip is positioned within the accommodation slots, the first end is positioned in correspondence with the outlet orifices; and
the test strip comprises a backing plate, and a sample pad, a colloidal gold pad, a nitrocellulose membrane, and an absorbent pad sequentially disposed on the backing plate along a direction extending from the first end to the second end, wherein
the nitrocellulose membrane is coated with a plurality of test lines and a control line.

15. The multiplex testing apparatus of claim 14, wherein the nitrocellulose membrane is positioned in correspondence with the observation windows.

16. The multiplex testing apparatus of claim 14, wherein the plurality of test lines are coated with multiple types of allergen antigens, while the control line is coated with goat anti-chicken IgY antibodies; and
each of the test lines is coated with a distinct one of the multiple types of allergen antigens.

17. The multiplex testing apparatus of claim 16, wherein the multiple types of allergen antigens comprise inhaled allergen antigens and ingested allergen antigens, wherein
the inhaled allergen antigens comprise one selected from the group consisting of: cat dander, mugwort, birch trees, timothy grass, cockroaches, dog dander, plantains, ragweed, house dust mites, and *Alternaria alternata;* and
the ingested allergen antigens comprise one selected from the group consisting of: peanuts, milk, codfish, scallops, chicken egg white, shrimp, crabs, soybeans, wheat, and almonds.

18. The multiplex testing apparatus of claim 14, wherein antibodies coated on the colloidal gold pad are colloidal gold particle-labeled mouse anti-human IgE antibodies and colloidal gold particle-labeled chicken IgY antibodies.

19. A use method of a multiplex testing apparatus, the multiplex testing apparatus comprising a test card and a test strip, wherein the test card is provided with a sample loading port, and the use method comprises:
loading a test sample into the sample loading port; and
determining test information generated by the test strip in response to the test sample.

20. The use method of the multiplex testing apparatus of claim 19, wherein the test sample is a whole blood sample, a plasma sample, or a serum sample.
